Europäisches Patentamt

European Patent Office

Office européen des brevets

(19)

(11) Publication number: **0 256 421**
**A2**

# EUROPEAN PATENT APPLICATION

(21) Application number: **87111245.4**

(22) Date of filing: **04.08.87**

(51) Int. Cl.⁴: **C12N 15/00** , **C12P 21/00** , **C12N 1/16** , **C07H 21/04**

30 additional claims annexed to the specification are deemed to be abandoned due to non-payment of the claims fees (Rule 31 (3) EPC).

The applicant has filed a statement in accordance with Rule 28 (4) EPC (issue of a sample only to an expert). Accession number(s) of the deposit(s): NRRL Y 11430/1, 15851/68/90, 18014/5/7/21/83.

(30) Priority: **12.08.86 US 895632**

(43) Date of publication of application:
**24.02.88 Bulletin 88/08**

(84) Designated Contracting States:
**AT BE CH DE ES FR GB IT LI LU NL SE**

(71) Applicant: **PHILLIPS PETROLEUM COMPANY**
**5th and Keeler**
**Bartlesville Oklahoma 74004(US)**

(72) Inventor: **Tschopp, Juerg Friedrich**
**102458 Carmel Cape**
**San Diego CA 92130(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) Secretion of heterologous proteins from yeast.

(57) Method for production by yeast of secreted proteins having a pattern of glycosylation substantially similar to the high mannose-type mammalian protein pattern of glycosylation, and DNA sequences useful therefor are described. In addition, method for rendering yeast strains competent for growth on carbon and energy sources on which prototrophic strains of yeast are unable to grow is provided.

**FIG. 5**

EP 0 256 421 A2

## SECRETION OF HETEROLOGOUS PROTEINS FROM YEAST

This invention relates to the field of recombinant DNA technology. In one of its aspects, the present invention relates to the secretion of heterologous proteins. In another aspect, the present invention relates to the growth of yeast strains on carbon sources on which prototrophic strains of such yeasts are incapable of growing.

### BACKGROUND

Commercial efforts employing recombinant DNA technology for producing various polypeptides have, up to now, centered predominantly on *Escherichia coli* as a host organism. However, in some situations *E. coli* may prove to be unsuitable as a host. For example, *E. coli* contains a number of toxic pyrogenic factors that must be eliminated from any polypeptide useful as a pharmaceutical product. The efficiency with which this purification can be achieved will, of course, vary with the particular polypeptide. In addition, the proteolytic activities of *E. coli* can seriously limit yields of some useful products. Moreover, proteins expressed at high levels in *E. coli* are frequently packaged into inclusion bodies and are difficult to solubilize. Thus recovery of biologically active proteins from *E. coli* can, at times, be troublesome. The difficulties encountered in recovering *E. coli* produced proteins, as well as the low biological activity of such proteins may be the result of the inability of *E. coli* to carry out certain post-translational processes, e.g., glycosylation of the recombinant DNA produced protein. These and other considerations have led to increased interest in alternative hosts, in particular, the use of eukaryotic organisms for the production of polypeptide products is appealing.

The availability of means for the production of polypeptide products in eukaryotic systems, e.g., yeast, could provide significant advantages relative to the use of prokaryotic systems such as *E. coli* for the production of polypeptides encoded by recombinant DNA. Yeast have been employed in large scale fermentations for centuries, as compared to the relatively recent advent of large scale *E. coli* fermentations. Yeast can frequently be grown to higher cell densities than bacteria and are readily adaptable to continuous fermentation processing. In fact, growth of yeast such as *Pichia pastoris* to ultra-high cell densities, i.e., cell densities in excess of 100 g/L, is disclosed by Wegner in U.S. 4,414,329 (assigned to Phillips Petroleum Co.).

Additional advantages of yeast hosts include the fact that many critical functions of the organism, e.g., oxidative phosphorylation, are located within organelles, and hence are not exposed to the possible deleterious effects of the organism's production of polypeptides foreign to the wild-type host cells. An alternate method for avoiding such deleterious effects is to produce the heterologous protein, i.e., the foreign protein produced employing recombinant DNA techniques, in secreted form. Secretion of the heterologous protein not only has the advantage of reduced impact on the host cell, additional benefits of secretion include the ease of product purification, the increased stability of proteins when glycosylated and the reduced exposure of the desired product to protein degrading enzymes in the cell. It is also possible that as a eukaryotic organism, yeast will exhibit the same codon preferences as higher organisms, thus tending toward more efficient production of expression products from mammalian genes or from complementary DNA (cDNA) obtained by reverse transcription from, for example, mammalian mRNA.

The ability of yeasts to glycosylate expressed polypeptide products, as well as the degree and pattern of such glycosylation, are also important considerations when such glycosylation is important to the bioactivity of the polypeptide product. The ability to glycosylate polypeptide products expressed employing recombinant DNA techniques in a consistent manner would be highly desirable.

One potential drawback to the use of yeast for certain conversion processes is that the range of carbon and energy sources on which a number of yeast strains grow are, unfortunately, limited. For example, strains of *Pichia pastoris* are not sucrose or lactose competent, and thus are unable to grow on these very readily available, inexpensive carbon sources. As another example, strains of *Saccharomyces cerevisiae* are lactose incompetent, and are also unable to grow on this readily available, inexpensive carbon source. Thus, the ability to render a variety of strains of yeast competent for growth on inexpensive carbon and energy sources would be highly desirable.

## OBJECTS OF THE INVENTION

An object of the present invention, therefore, is the development of methods for secreting proteins having high mannose-type mammalian protein pattern of glycosylation from recombinant DNA modified yeast.

Another object of the present invention is the development of methods for the growth of yeast strains on substrates which the wild-type yeast strains are incapable of assimilating as a carbon and energy source.

Yet another object of the present invention is to provide novel yeast strains which are competent for growth on carbon and energy sources which the wild-type yeast strains are incapable of utilizing for growth.

Still another object of the present invention is to provide novel DNA fragments useful for the controlled expression and secretion of heterologous proteins by yeast.

These and other objects of the invention will become apparent from inspection of the disclosure, drawings and claims herein provided.

## STATEMENT OF THE INVENTION

In accordance with the present invention, there has been developed a method for producing proteins having a high mannose-type mammalian protein pattern of glycosylation by expressing a nucleic acid sequence coding for the desired protein in a host cell of the genus *Pichia* under the control of a yeast promoter and a yeast secretion signal.

The proteins produced in accordance with the invention have glycosylation patterns much more like the glycosylation patterns of mammalian proteins than do proteins made by recombinant DNA techniques employing other non-mammalian host systems, and thus are more likely to have similar physical, chemical and biological properties relative to naturally occurring proteins. In addition, since the glycosylated products of the present invention are produced in secreted form, product purification is greatly simplified relative to product isolation where heterologous proteins are produced as intracellular materials rather than as extracellular materials.

Further in accordance with the present invention, there has been developed a method for rendering yeast strains competent for growth on carbon and energy sources which the wild-type yeast strains are incapable of utilizing for growth, which method comprises first transforming yeast with a novel DNA fragment coding for expression and secretion of the gene function which the wild-type yeast lacks, regenerating the transformants on non-selective medium, and then subjecting the regenerated transformants to selective growth conditions wherein only those colonies which have incorporated the novel DNA fragment used for transformation are capable of growth. The transformants selected in accordance with the previous step are capable of growth on the substrate which the wild-type yeast strains would otherwise be incapable of utilizing for growth, absent the introduction of the above described DNA fragment and expression and secretion of the gene product.

The novel strains produced in accordance with this aspect of the invention are capable of growth on readily available, inexpensive, carbon sources.

## BRIEF DESCRIPTION OF THE FIGURES

Figure 1 is a restriction map of the primary *Pichia* dihydroxyacetone synthase gene (DAS1) regulatory region.

Figure 2 is a restriction map of the primary *Pichia* alcohol oxidase gene (AOX1) regulatory region.

Figure 3 is a restriction map of the *Pichia* p40 gene regulatory region.

Figure 4 is a linearized restriction map of the closed circular plasmid p/TSU1.

Figure 5 is a schematic representation of the steps employed to construct plasmids pGS101 and pGS102.

Figure 6 is a restriction map of plasmid pGS101.

Figure 7 is a restriction map of plasmid pGS102.

The following abbreviations are used in the Figures to represent the restriction enzymes employed:

A = *Asu*II

B = *Bam*HI

B₂ = *Bgl*II

Bc = *Bcl*I

3

C = ClaI
H₂ = HincII
H₃ = HindIII
K = KpnI
Nd₁ = NdeI
Ps = PstI
Pv₁ = ·PvuI
Pv₂ = PvuII
R₁ = EcoRI
R₅ = EcoRV
S = SalI
Ss = SstI

## DETAILED DESCRIPTION OF THE INVENTION

In accordance with one aspect of the present invention, proteins having a pattern of glycosylation substantially similar to the high mannose-type mammalian protein pattern of glycosylation are produced in secreted form in yeast by expressing a coding sequence coding for the desired protein in a host cell of the genus *Pichia* under the control of a promoter and a yeast secretion signal, e.g., the *Saccharomyces cerevisiae* invertase gene secretion signal.

Proteins contemplated for expression in accordance with the present invention include those proteins which are characterized by glycosylation in a pattern similar to that of high mannose-type mammalian proteins. Such pattern typically involves the bonding of an average of 5 up to 9 mannose units per glycosylation site, and generally at least about 71%, but not greater than 82% of the sugar units employed for glycosylation at each glycosylation site are mannose sugar units. In accordance with the present invention, it has been found that the glycosylation pattern imparted by *Pichia* typically involves the bonding of an average of 9 up to 14 mannose units per glycosylation site, and generally at least about 80%, but not greater than 88% of the sugar units employed for glycosylation at each glycosylation site are mannose sugar units. Thus, due to the substantial similarity of the glycosylation pattern imparted by *Pichia* compared to the high mannose-type mammalian pattern of glycosylation, proteins glycosylated by *Pichia* are expected to have both physical structure and biological activity which closely resemble that of authentic proteins having the high mannose-type mammalian pattern of glycosylation.

Exemplary proteins which fall within the above definition of "high mannose-type" mammalian pattern of glycosylation include:

HTLV-III env protein,
chicken ovalbumin,
Sindbis virus proteins,
bovine rhodopsin,
human immunoglobulins, and
viral antigens, and the like.

Exemplary strains of *Pichia* contemplated within the scope of the present invention include *Pichia pastoris* NRRL Y-11430, *Pichia pastoris* NRRL Y-11431, *Pichia pastoris* NRRL Y-15851, *Pichia pastoris* NRRL Y-18014, *Pichia pastoris* NRRL Y-18017, and the like.

Any regulatory region which functions to regulate gene expression in the yeast host is suitable for use in the practice of the present invention. The term "regulatory region" as used in this disclosure is intended to include the various functions necessary for controlled gene expresison, e.g., promoter regions, upstream activator sequences, catabolite sensitive regions, and the like. Those of skill in the art are aware of numerous regulatory regions which have been characterized and which could be employed in the practice of the present invention.

Exemplary regulatory regions which regulate gene expression in yeast include, but are not limited to: the acid phosphatase, invertase, alpha-mating factor and glyceraldehyde 3-phosphate dehydrogenase regulatory regions isolated from *Saccharomyces cerevisiae*; the primary alcohol oxidase (AOX1) and dihydroxyacetone synthase (DAS1) and p40 regulatory regions isolated from *Pichia pastoris*; the *Pichia* HIS4 regulatory region; and the like.

Presently preferred regulatory regions employed in the practice of the present invention are characterized by their ability to respond to media-containing:

(1) methanol,

(2) non-catabolite representing carbon sources such as, for example, glycerol, galactose, acetate, and the like,

(3) catabolite repressing carbon sources, such as, for example, glucose, ethanol, fructose, and the like, followed by carbon source starvation.

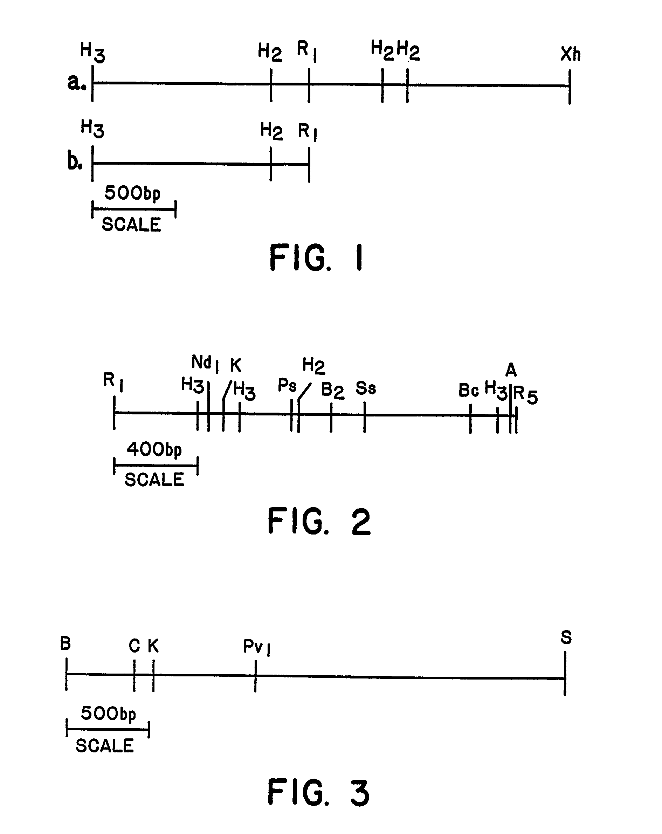

Examples of these presently preferred regulatory regions are depicted by the restriction maps set forth in Figures 1, 2 and 3. The regulatory region depicted in Figure 1 is derived from the primary dihydroxyacetone synthase (DAS1) gene of *Pichia pastoris*. The regulatory region depicted in Figure 2 is derived from the primary alcohol oxidase (AOX1) gene of *Pichia pastoris* (*Pichia* has two alcohol oxidase genes, referred to hereinafter as AOX1 and AOX2). The regulatory region depicted in Figure 3 is derived from the p40 gene of *Pichia pastoris*. Those of skill in the art recognize that other regulatory regions having the above-described properties can be isolated from methylotrophic yeasts, such as for example, *Pichia pastoris*. Such additional regulatory regions having regulatory properties similar to the properties of the above-described regulatory regions are also within contemplation of the present invention.

Exemplary yeast secretion signals include the invertase, acid phosphatase secretion signals isolated from *Saccharomyces cerevisiae* ; the acid phosphatase secretion signal isolated from *Pichia pastoris*; and the like.

The presently preferred secretion signal for use in the practice of the present invention is the invertase gene secretion signal. This preferred secretion signal employed in the practice of the present invention is any gene which encodes the following amino acid sequence:

Met-Leu-Leu-Gln-Ala-Phe-Leu-Phe-Leu-Leu-Ala-Gly-Phe-Ala-Ala-Lys-Ile-Ser-Ala-.

One such nucleotide sequence is represented by the following 57 base pair fragment:

ATG-CTT-TTG-CAA-GCT-TTC-CTT-TTC-CTT-TTG-GCT-GGT-TTT-GCA-GCC-AAA-ATA-TCT-GCA-.

The selected promoter, secretion signal and the desired coding sequence are ligated together in serial sequence employing techniques known by those of skill in the art to produce novel DNA fragments. These novel DNA fragments are used to transform host strains of the genus *Pichia* , which then express the desired protein in secreted form and having the desired high mannose-type mammalian protein pattern of glycosylation when the transformed host cells are subjected to conditions under which the promoter employed is activated.

In accordance with another embodiment of the present invention, yeast cells are rendered competent for growth on carbon and energy sources which the yeast cells cannot normally metabolize by providing to the host yeast cell the gene functions which are required for metabolism of the desired carbon and energy source.

The host yeast cells employed in accordance with this embodiment of the present invention can be either prototrophic or auxotrophic strains. The advantage of utilizing prototrophic strains is that they are readily available, and frequently have better growth properties than do auxotrophic strains. On the other hand, auxotrophic strains allow for the selection of transformants and continued application of selection pressure on the transformants by complementation of the missing gene function.

The gene functions in which some strains of the genus *Pichia* are deficient include those involved in the utilization of such carbon and energy sources as sucrose (i.e., the invertase gene), mellibiose (i.e., the alpha-galactosidase gene), lactose (i.e., the beta-galactosidase and lactose permease genes), maltose (i.e., the beta-glucosidase gene) and the like.

The experimental procedures for the transformation of *Pichia pastoris* have been previously described (Cregg et al., Mol. & Cell. Biol., vol. 5, pp. 3376-3385 (1985)), and are presented in greater detail below (Example I). The experimental procedure for the transformation of *Saccharomyces cerevisiae* has been previously described by Ito et al. (J. Bacteriol., vol. 153, pp. 163-168 (1983).

The invention will now be described in greater detail with reference to the following non-limiting examples.

EXAMPLES

The following reagents and media are used throughout the following examples:

SED

   2 M Sorbitol
   0.2 M EDTA
   1 M DTT
   --adjust to pH 8

SCE

    2 $\underline{M}$ Sorbitol

    1 $\underline{M}$ Sodium citrate

    2 $\underline{M}$ EDTA

CaS

    1 $\underline{M}$ Sorbitol

    1 $\underline{M}$ Tris-HCl (pH 7.5)

    1 $\underline{M}$ CaCl$_2$

PEG Solution

    2 g. PEG 4000

    --bring total volume to 10 mL. with H$_2$O

SOS

    1 $\underline{M}$ Sorbitol

    0.3 X YPD medium

    10 mM CaCl$_2$

Laemmli Loading Buffer

    62.5 m$\underline{M}$ Tris-HCl (pH 6.8)

    2% SDS

    10% glycerol

    5% 2-mercaptoethanol

    0.01% bromphenol blue

Invertase Assay Mix

    0.08 $\underline{M}$ Sodium acetate, pH 5.1

    0.1 $\underline{M}$ Sucrose

    0.2% Triton X-100

Glucose Oxidase Reagent

    3.0 U/mL glucose oxidase

    2.5 $\mu$9l/mL peroxidase

    0.15 mg/mL o-dichlorobenzene

    0.1 m$\underline{M}$N-ethylmaleimide

    0.01 $\underline{M}$ potassium phosphate, pH 7.0

YPD

    Yeast extract (10 g), peptone (20 g), and dextrose (10 g) per liter.

MD

    Yeast nitrogen base without amino acids (YM, 13.4 g), dextrose (10 g), and biotin (400 $\mu$9l) per liter.

MSS

    YM (13.4 g), sucrose (10 g), and biotin (400 $\mu$9l) per liter.

<u>Fermentation</u> <u>Media</u>  (mg% is the number of milligrams of a given component per 100 mL of total media.)

All strains were maintained on $\underline{MM}$ minimal <u>medium</u>: (KH$_2$PO$_4$, 87.5 mg%; K$_2$HPO$_4$, 12.5 mg%, (NH$_4$)$_2$SO$_4$, 100 mg%; MgSO$_4$•7H$_2$O, 50 mg%, NaCl, 10 mg%; CaCl$_2$•H$_2$O, 10 mg%;-FeCl$_3$•6H$_2$O, 5 ng%; ZnSO$_4$•7H$_2$O, 7 ng%, H$_3$BO$_4$, 1 ng%; CuSO$_4$•5H$_2$O, 1 ng%; KI, 1 ng%), pH 5.8 supplemented with 1% glucose or 1% sucrose, 5 ng% biotin, and 2 mg% histidine, as required.

Chemostat cultures were started in two liters of 1X <u>FM</u> <u>-21</u> minimal <u>medium</u>: (85% H$_3$PH$_4$, 3.5 mL/100 mL; CaSO$_4$•2H$_2$O, 15 mg%; K$_2$SO$_4$, 238 mg%; MgSO$_4$•7H$_2$O, 195 mg%; KOH, 65 mg%; FeSO$_4$•7H$_2$O, 6.5 ng%; CuSO$_4$•5H$_2$O, 6 mg%; ZnSO$_4$•7H$_2$O, 20 mg%; MnSO$_4$•H$_2$O, 3 mg%; biotin, 4.1 ng%; DOW Corning FG-10 antifoam, 8 drops/liter supplemented with 2% glucose or sucrose as required and brought to and maintained at pH 4.0 with NH$_3$ gas.

## VECTORS

The *SUC2* gene from *Saccharomyces cerevisiae* has been isolated and sequenced, as disclosed by Carlson and Botstein in NAR 11:1943 (1983). The position of the promoter, signal sequence and structural gene has been delineated and are described by Carlson and Botstein.

The alcohol oxidase (AOX1) gene and its promoter have been isolated and are described in Ellis et al. in Mol. Cell. Biol. 5:1111 (1985). A restriction map of the AOX1 promoter is presented in Figure 2.

The *Pichia* and *Saccharomyces* invertase-based secretion vectors discussed in this application were assembled using the SUC2 and AOX1 sequences described above, as well as various other components necessary for vector maintenance and selection.

## EXAMPLE I

### Yeast Transformation Procedures

#### I. *Pichia pastoris*

##### A. Cell Growth

1. Inoculate a colony of *Pichia pastoris* GS115 (NRRL Y-15851) into about 10 mL of YPD medium and shake culture at 30°C for 12-20 hrs.

2. After about 12-20 hrs., dilute cells to an $OD^{600}$ of about 0.01-0.1 and maintain cells in log growth phase in YPD medium at 30°C for about 6-8 hrs.

3. After about 6-8 hrs, inoculate 100 mL of YPD medium with 0.5 mL of the seed culture at an $OD^{600}$ of about 0.1 (or equivalent amount). Shake at 30°C for about 12-20 hrs.

4. Harvest culture when $OD^{600}$ is about 0.2-0.3 (after approximately 16-20 hrs) by centrifugation at 1500 g for 5 minutes.

##### B. Preparation of Spheroplasts

1. Wash cells once in 10 mL of sterile water. (All centrifugations for steps 1-5 are at 1500 g for 5 minutes.)

2. Wash cells once in 10 mL of freshly prepared SED.

3. Wash cells twice in 10 mL of sterile 1 $\underline{M}$ Sorbitol.

4. Resuspend cells in 10 mL SCE buffer.

5. Add 5-10 μL of 4 mg/mL Zymolyase 60,000 (Miles Laboratories). Incubate cells at 30°C for about 30-60 minutes.

Since the preparation of spheroplasts is a critical step in the transformation procedure, one should monitor spheroplast formation as follows: add 100 μL aliquots of cells to 900 μL of 5% SDS and 900 μL of 1 $\underline{M}$ Sorbitol before or just after the addition of zymolyase and at various times during the incubation period. Stop the incubation at the point where cells lyse in SDS but not in sorbitol (usually between 30 and 60 minutes of incubation).

6. Wash spheroplasts twice in 10 mL of sterile 1 $\underline{M}$ Sorbitol by centrifugation at 1000 g for 5-10 minutes. (The time and speed for centrifugation may vary; centrifuge enough to pellet spheroplasts but not so much that they rupture from the force.)

7. Wash cells once in 10 mL of sterile CaS.

8. Resuspend cells in total of 0.6 mL of CaS.

##### C. Transformation

1. Add DNA samples (up to 20 μL volume) to 12 × 75 mm sterile polypropylene tubes. (DNA should be in water or TE buffer; for maximum transformation frequencies with small amounts of DNA, it is advisable to add about 1 μL of 5 mg/mL sonicated *E. coli* DNA to each sample.)

2. Add 100 μL of spheroplasts to each DNA sample and incubate at room temperature for about 20 minutes.

3. Add 1 mL of PEG solution to each sample and incubate at room temperature for about 15 minutes.

4. Centrifuge samples at 1000 g for 5-10 minutes and decant PEG solution.

5. Resuspend samples in 150 $\mu$L of SOS and incubate for 30 minutes at room temperature.

6. Add 850 $\mu$L of sterile 1 M Sorbitol and plate aliquots of samples as described below.

### D. Regeneration of Spheroplasts

1. Recipe for Regneration Agar Medium:

a. Agar-Sorbitol-9 g Bacto-agar, 54.6 g Sorbitol, 240 mL H$_2$O, autoclave.

b. 10X Glucose-20 g Dextrose, 100 mL H$_2$O, autoclave.

c. 10X SC-6.75 g Yeast Nitrogen Base without amino acids, 100 mL H$_2$O, autoclave. (Add any desired amino acid or nucleic acid up to a concentration of 200 $\mu$9l/mL before or after autoclaving.)

d. Add 30 mL of 10X Glucose and 30 mL of 10X SC to 300 mL of the melted Agar-Sorbitol solution. Add 0.6 mL of 0.2 mg/mL biotin and any other desired amino acid or nucleic acid to a concentration of 20 $\mu$9l/mL. Hold melted Regeneration Agar at 55-60°C.

2. Plating of Transformation Samples:

Pour bottom agar layer of 10 mL Regneration Agar per plate at least 30 minutes before transformation samples are ready. Distribute 10 mL aliquots of Regneration Agar to tubes in a 45-50°C bath during the period that transformation samples are in SOS. Add aliquots of transformation samples described above to tubes with Regeneration Agar and pour onto bottom agar layer of plates. Add a quantity of each sample to 10 mL aliquots of melted Regeneration Agar held at 45-50°C and pour each onto plates containing a solid 10 mL bottom agar layer of Regeneration Agar.

3. Determination of Quality of Spheroplast Preparation:

Remove 10 $\mu$L of one sample and dilute 100 times by addition to 990 $\mu$L of 1 M Sorbitol. Remove 10 $\mu$L of the 100 fold dilution and dilute an additional 100 times by addition to a second 990 $\mu$L aliquot of 1 M Sorbitol. Spread plate 100 $\mu$L of both dilutions on YPD agar medium to determine the concentration of unspheroplasted whole cells remaining in the preparation. Add 100 $\mu$L of each dilution to 10 mL of Regeneration Agar supplemented with 40 $\mu$9l/mL histidine to determine total regeneratable spheroplasts. Good values for a transformation experiment are 1-3 $\times$ 10$^7$ total regeneratable spheroplasts/mL and about 1 $\times$ 10$^3$ whole cells/mL.

4. Incubate plates at 30°C for 3-5 days.

## II. Saccharomyces cerevisiae

Transformations of S. cerevisiae were carried out according to the procedure of Ito et al. (J. Bacteriol., vol. 153, pp. 163-168 (1983).

## EXAMPLE II

### VECTOR CONSTRUCTION

a. Vector pTSU1 (Pichia)

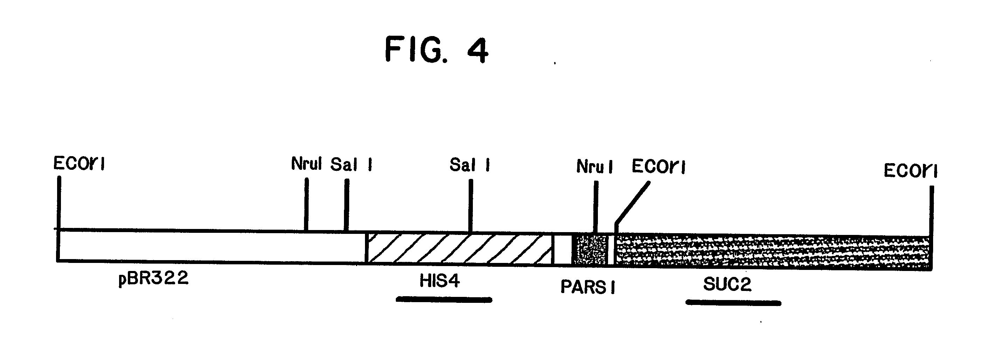

Figure 4 depicts a linearized restriction map of the Pichia vector pTSU1 which is comprised of the promoter, signal, and structural portions of the S. cerevisiae SUC2 gene. The three components of the expression cassette employed, i.e., the promoter, the signal sequence and structural gene are sometimes hereinafter designated by the shorthand: Promoter-Signal-Structural gene. Thus the construction employed in pTSU1 is designated SUC2-SUC2-SUC2. Plasmid pTSU1 was derived from the readily available plasmid pRB58 by cutting with EcoR1 and isolating the entire SUC2 gene. Ligation of the EcoR1-ended SUC2 gene with the EcoR1-cut pYJ30 plasmid (available in an E. coli host from the USDA, Northern Regional Research Center in Peoria, Illinois, under the accession number NRRL B-15890) yielded vector pTSU1, which is ampicillin resistant, has the Pichia autonomous replication aequence PARS1, and has the P. pastoris HIS4 gene for selection in His4$^-$ Pichia cells. Plasmid pTSU1, in an E. coli host, has been deposited with the Northern Regional Research Center of the United States Department of Agriculture in Peoria, Illinois, in order to ensure access to this plasmid by the public upon issuance of this application as a patent. This strain has the laboratory designation HB101/pTSU1, and has been assigned accession number NRRL B-18083.

### b. Vector pGS102 (Pichia) and pGS101 (Saccharomyces)

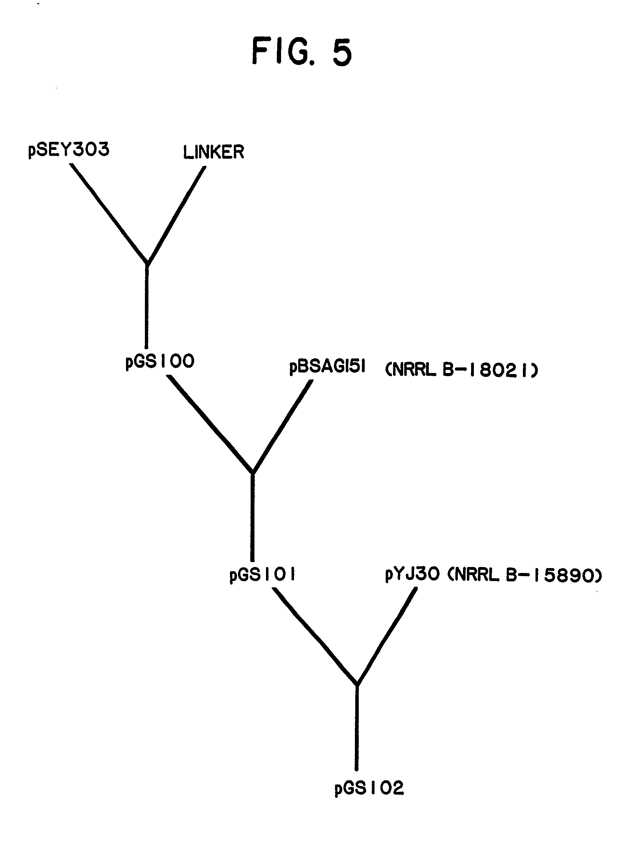

The Pichia pastoris AOX1 promoter element, which is regulatable by methanol, is used to promote expression of the SUC2 signal-SUC2 structural gene in the expression cassette AOX1-SUC2-SUC2. Figure 5 outlines the steps in the construction of vector pGS102. Since the SUC2 gene in the widely available plasmid pSEY303 has only a partial signal sequence, followed by the complete SUC2 structural gene, a linker was made to add back the missing signal sequence by allowing two synthetic oligonucleotides to hybridize, giving the following sequence:

$$5'-A\ A\ T\ T\ C\ A\ T\ G\ A\ T\ G\ C\ T\ T\ C\ T\ T\ C\ A-3'$$
$$3'-G\ T\ A\ C\ T\ A\ C\ G\ A\ A\ G\ A\ A\ G\ T\ T\ C\ G\ A-5'$$

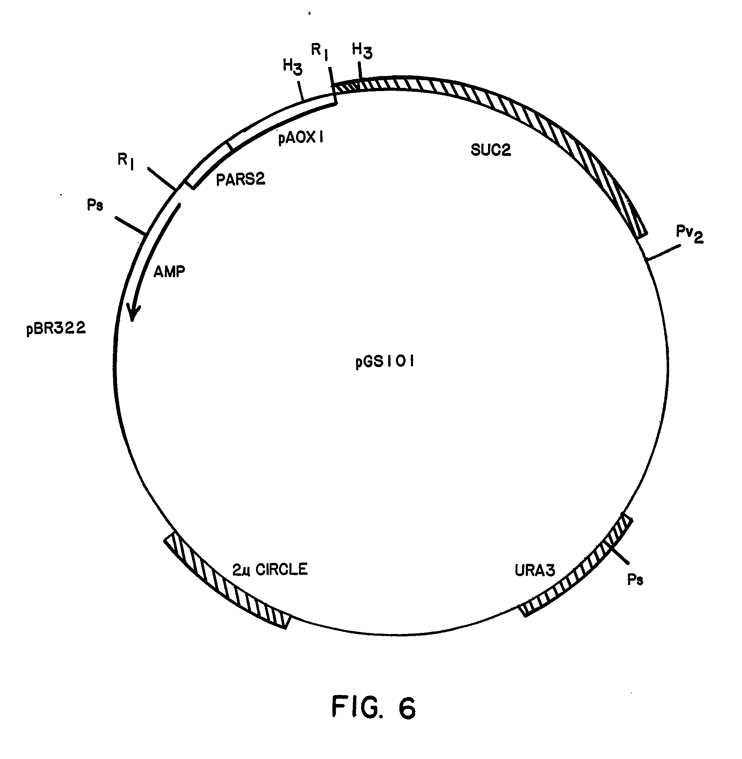

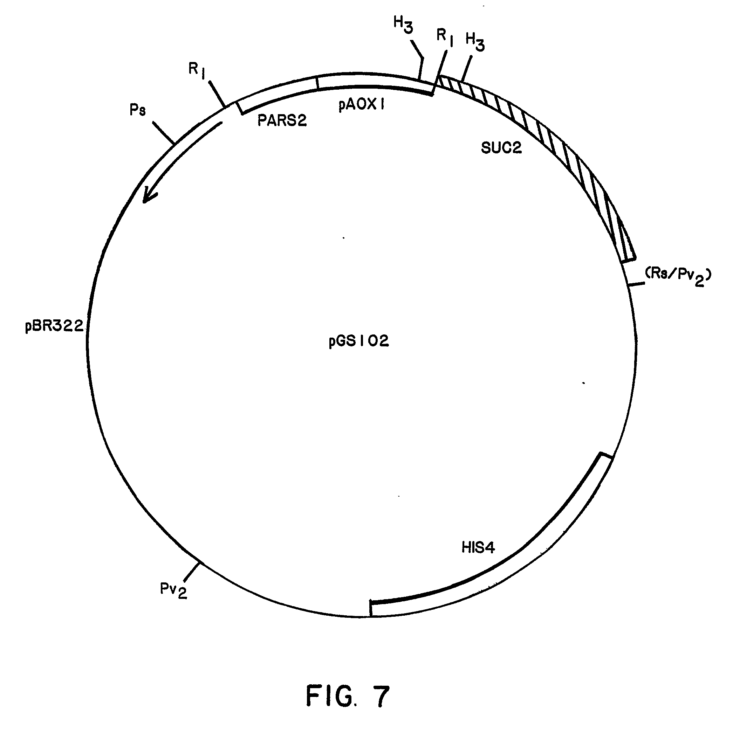

The resulting vector pGS100 has the complete SUC2 signal-structural gene and, when opened at EcoR1 and ligated to the EcoR1 fragment from pBSAGI5I (available in an E. coli host from the USDA, Northern Regional Research Center in Peoria, Illinois, under the accession number NRRL B-18021) which contains the PARS1 and AOX1 promoter elements, forms vector pGS101. Vector pGS101 was used to transform Saccharomyces cells. Ligation of the large Pst I-EcoRV fragment from pYJ30 (available in an E. coli host from the USDA, Northern Regional Research Center in Peoria, Illinois, under the accession number NRRL B-15890) into pGS101 yielded pGS102, which was used to transform Pichia. Detailed restriction maps of pGS101 and pGS102 are shown in Figures 6 and 7, respectively.

Suitable hosts for transformation with these vectors include:

Pichia GS115 (NRRL Y-15851)
Pichia KM71
Saccharomyces SEY2102
Saccharomyces SEY5188 (secretory mutant)

Pichia GS115 is a his4 defective mutant of Pichia pastoris, available from the USDA, Northern Regional Research Center in Peoria, Illinois, under the accession number NRRL Y-15851.

Pichia KM71 is a Pichia strain having the genotype: ( his4 aox1::SARG4). This strain was created by transforming Pichia PPF1 (a his4 arg 4 double auxotrophic mutant, available from the USDA, Northern Regional Research Center in Peoria, Illinois, under the accession number NRRL Y-18017) with a linearized portion of plasmid pYMI7 (which contains the Saccharomyces ARG4 gene flanked by 3'-and 5'-Pichia alcohol oxidase gene (AOX1) sequences). Plasmid pYMI7 was constructed by inserting a 2.9 kbp BamHI-SaII fragment from plasmid pYM25 (available in an E. coli host from the USDA, Northern Regional Research Center in Peoria, Illinois, under the accession number NRRL B-18015, which plasmid contains the Saccharomyces ARG4 gene) into BamHI-cut pPG4.0 (available in an E. coli host from the USDA, Northern Regional Research Center in Peoria, Illinois, under the accession number NRRL B-15868). The insertion results in a deletion of about 600 bp from the 5'-portion of the AOX1 gene (about one fourth of the gene). Plasmid pYMI7 was linearized by digestion with PvuII and EcoRI and transformed into PPF1 (arg4 his4, NRRL Y-18017) by selecting for Arg+ prototrophs. Transformants were extracted from the regeneration agar, sonicated and spread on SD medium agar plates containing 0.1% glucose and 40 μgl/mL histidine. Colonies which resulted were then replica plated onto a set of SD medium agar plates (containing histidine) with the following carbon sources: 1) no carbon; 2) 0.5% methanol; and 3) 2% glucose. About 81.0% of the Arg+ colonies could not grow normally on methanol. Southern blot analysis of genomic DNA from one of the methanol nonutilizers confirmed that the AOX1 gene was disrupted in the strain designated as KM71.

Saccharomyces strains SEY2102 and SEY5188 are both widely distributed, and hence readily available.

### EXAMPLE III

## INVERTASE SECRETION

### a. Culture Growth Conditions

*P. pastoris* cells were grown in yeast nitrogen base (YM) supplemented with glucose, glycerol, or methanol as the carbon source. Transformants were inoculated from a plate in YM supplemented with 2% glucose or 3% glycerol at a cell density of 0.02 to 0.05 OD$^{600}$ units. Cells were grown to mid-log phase (0.5 to 1.0 OD$^{600}$ units) in shake flasks (500 mL) containing 100 to 200 mL medium. Prior to a shift to methanol medium, cells from a glycerol culture were centrifuged, washed once with sterile water and resuspended at 0.05 to 0.1 OD$^{600}$ units in 100 to 200 mL of 0.5% methanol in YM (500 mL shake flasks). *P. pastoris* transformants containing vectors pGS102 or pTSU1 were usually grown in methanol medium prior to analysis of cells and culture medium. Host cells absent the plasmid DNA were grown under the same conditions except that histidine (20 mg/L) was added to the culture medium.

*S. cerevisiae* cells were grown in Yeast Nitrogen Base (YM) supplemented with glucose and leucine (30 mg/L) and histidine (20 mg/L) or yeast extract and peptone (YP) supplemented with glycerol. Transformants were inoculated from a plate in YMB supplemented with 5% glucose and leucine and histidine (20 to 30 mg/L) at a cell density of 0.05 to 0.1 OD$^{600}$ units. Cells were grown to mid-log phase (0.5 to 1.0 OD$^{600}$ units) in shake flasks (500 mL) containing 100 to 200 mL medium. Prior to a shift in YM containing 0.1% glucose or YP containing 3% glycerol the cells were washed once with distilled water. *S. cerevisiae* transformants containing vector pRB58 were usually shifted to 0.1% glucose medium to allow derepression for invertase prior to analysis of the cells. Transformants containing vector pGS101 were usually shifted to glycerol medium to activate the alcohol oxidase promoter to produce invertase. Host cells absent the plasmid DNA were grown under the same conditions except that uracil (20 mg/L) was added to the growth medium. *S. cerevisiae* secretory mutants were grown at 24°C (instead of 30°C) prior to the shift in low glucose medium.

### b. Invertase Assay

0.1 to 1 mL of culture was centrifuged in an Eppendorf centrifuge. The cell-free growth medium was carefully removed from the cell pellet and 10-100 μl was used for the invertase assay (secreted invertase). The cell pellet was resuspended in water at a cell density of 0.02-0.2 OD$^{600}$ units. 50 μl of this suspension, or 1-10 milli-OD$^{600}$ units of cells, was used for the invertase assay (cellular invertase). 0.25 mL of assay mixture was added to the cell suspension or medium aliquot and incubated at 37°C for 10-30 minutes. The reaction was stopped with 0.3 mL of 0.2 M K$_2$HPO$_4$ and samples were boiled for 5 minutes in a boiling water bath. After cooling on ice, 2 ml of glucose oxidase reagent was added to the assay and the incubation was continued at 30°C for 30 minutes. The reaction was stopped with 2 mL 6N HCl and the absorbance was read at 540 nm. In parallel, aliquots of 10-50 μl of 1 mg/mL of glucose were included in the assay as a standard. Invertase units, U, are defined as that amount of enzyme which causes the release of 1 μ mole of glucose in one minute at 37°C, and are calculated according to the following formula:

$$1U = \frac{A^{540} \text{ (sample)}}{A^{540} \text{ (50 μL standard)}} \quad X \quad \frac{1}{\text{time (min)}} \quad X \ 0.278$$

## C. Results

The invertase units measured in the various transformants, grown as described above, are summarized in the following Table.

### TABLE

| Host(1) | Strain | Genotype | Vector | Expression unit(2) | Carbon Source | Invertase Units/OD$_{600}$(3) | |
|---|---|---|---|---|---|---|---|
| | | | | | | Cells | Medium |
| Pp | GS115 | his4$^-$/AOX1$^+$ | pGS102 | AOX-SUC-SUC | 3% glycerol 0.5% MeOH | 1.25-2 | 5.5-7.5 |
| Pp | KM71 | his4$^-$/AOX1$^-$ | pGS102 | AOX-SUC-SUC | 3% glycerol 0.5% MeOH | 0.75 | 1.5-2.5 |
| Pp | GS115 | his4$^-$/AOX1$^+$ | pISU1 | SUC-SUC-SUC | 3% glycerol 0.5% MeOH | 22-35 | 5-7.5 |
| Sc | SEY2102 | ura3$^-$/his4$^-$/leu2 | pGS101 | AOX-SUC-SUC | 5% glucose | 0 | nd(4) |
| Sc | SEY2102 | ura3$^-$/his4$^-$/leu2 | pGS101 | AOX-SUC-SUC | 3% glycerol | 5 | 0.5 |
| Sc | SEY2102 | ura3$^-$/his4$^-$/leu2 | pRB58 | SUC-SUC-SUC | 5% glucose | 0 | nd |
| Sc | SEY2102 | ura3$^-$/his4$^-$/leu2 | pRB58 | SUC-SUC-SUC | 0.1% glucose | 2.5 | ≤0.1 |

(1) Pp = *Pichia pastoris* host; Sc = *Saccharomyces cerevisiae* host

(2) Expression unit is abbreviated as "promoter-secretion signal-structural gene

(3) 1 Unit = 1 μmole glucose released in one minute at 37°C;
1 OD$_{600}$ unit ~ 5 X 10$^7$ cells

(4) nd = not determined

11

The results in the Table demonstrate that the expression of invertase in *Pichia* from plasmid pGS102 is controlled in a regulatable manner by the *Pichia* AOX1 promoter element. No invertase is synthesized in the presence of glycerol, while a substantial amount is made upon addition of methanol. In addition, the AOX1⁻*Pichia* host appears to be a better host for production and secretion of invertase than the Pichia AOX1⁺ host or any of the *Saccharomyces* transformants.

It is also of interest to note that significant levels of invertase are obtained in *Pichia* even under the control of the non-*Pichia*derived SUC2 promoter.


## EXAMPLE IV

### ANALYSIS OF RECOMBINANT INVERTASE

The following analyses were conducted on periplasmic invertase. In those instances when secreted invertase was analyzed also, the results were identical to those found with periplasmic invertase (results not shown).


### a. Western Blot Analysis and Endo H Treatment

10 OD$^{600}$ units of cells (~5 × 10$^8$ cells) were removed from the culture and centrifuged in an Eppendorf centrifuge. The growth medium was removed (secreted invertase) and the cell pellet was washed with 0.5 mL of 50 mM Tris•HCl pH 7.5. The washed cell pellet was resuspended in 0.1 mL of the same buffer. 3 μL of 1.4M β-mercaptoethanol and 400 units of Zymolyase (~12 μL of 4 mg/mL of Zymolyase) was added and the suspension was incubated at 37°C for 30 min. The lysate was centrifuged in an Eppendorf centrifuge for 10 min and the soluble extract was removed from the cell debris. 5-10 μL of a 10-fold diluted soluble extract (cellular invertase) was assayed for invertase activity according to the method described previously.

5-20 μL of the original soluble extract (or 1 U of invertase activity) was mixed with an equal amount of two times concentrated Laemmli loading buffer and heated in a boiling water bath for five minutes. The sample was loaded on a 7.5% acrylamide gel and subjected to electrophoresis according to the procedure described by Laemmli (Nature 227, pp 680-685 (1970)).

In some cases endoglycosidase H (Endo H) was used to remove asparagine-linked sugars in soluble extracts prior to electrophoresis. 10 μL of soluble extract (or 1U of invertase activity) was added to 10 μL of 0.1M NaAc, pH 5.1, 0.4% SDS, and 2.5μL of 1 mg/mL of bovine serum albumin (BSA) and the sample was heated for 3-5 min in a boiling water bath. The sample was cooled on ice, 2-4 μL of Endo H (0.15-0.3 μ9l) were added and incubation at 37°C was continued for 5 hours. Then 25 μL of two-times concentrated Laemmli Loading buffer was added and the sample was heated for 5 min in a boiling water bath prior to loading on a 7.5% acrylamide gel. Western blot analysis was done according to Towbin et al.-(Proc. Nat. Acad. Sci. USA, vol. 76, pp. 4350-4354 (1979). Invertase antisera, raised in rabbits against either deglycosylated *S. cerevisiae* invertase or purified *Pichia* produced invertase and 125I labeled protein A were used for detection.

The Western blot pattern reveals the following: *Pichia* strains transformed with vector pGS102 or pTSU1 demonstrate invertase proteins in the range of 75-80 KD in contrast to the *S. cerevisiae* produced invertase which is heterogeneous and has a molecular weight in the range of 100-140 KD. After treatment with Endo H, all invertases produced by all *Pichia* and *Saccharomyces* strains collapse to a 60 KD band. This implies that the invertase protein is the same in all hosts and it is the amount and/or type of glycosylation that differs and causes the different molecular weights.


### b. Invertase Activity Gels

Preparation of soluble extracts and treatment with Endo H was done as described for Western blot analysis, except that two times concentrated Laemmli Loading Buffer was not added.

0.2-0.5 Units of invertase (30-50 μL of lysate) were mixed with an equal amount of 25% glycerol with a trace of bromphenol blue and were applied to the gel for electrophoresis at 4°C. Native gel electrophoresis was performed on 4.5% polyacrylamide slabs according to Rodbard and Chrombach (Anal. Biochem. 40:95-134, 1971). Invertase was localized in gels after incubation in 0.1M sodium acetate, pH 5.1, containing 0.1M sucrose for 10-90 min at 37°C by the method of Gabriel and Wang (Anal. Biochem. 27:545-554, 1969).

C. Density Gradient Analysis

Equilibrium density gradient analyses were performed by a modification of the procedure of Abrams et al. (J. Bacteriol. 135:809-817). Cells derepressed or induced for invertase were collected by centrifugation in an Eppendorf centrifuge, washed once with one-half ($\frac{1}{2}$) volume of 0.1M sodium acetate, pH 3.8, and resuspended in the same buffer at 100-200 $OD^{600}$ units/mL. Cells were broken with glass beads by shaking on a vortex mixer for one minute with one minute intervals on ice. The cell slurry was removed from the glass beads and particulate material was removed by centrifugation in an Eppendorf centrifuge for 10 min. A portion of the soluble extract (2-10 U of invertase activity) was mixed with 3 g acetate buffer and 2.5 g CsCl. Samples adjusted to 7.14 g with acetate buffer were centrifuged at 37,000 rpm for 45 hours in a Beckman SW50.1 rotor. Tubes were punctured at the bottom and fractions were collected and assayed for invertase activities according to the method described previously. Density was determined with a refractometer.

The density curves show that the Pichia -produced invertases have a buoyant density similar to the Saccharomyces sec18 mutant. The invertase produced by the wild-type Saccharomyces host is more heterogeneous and has a higher density (~1.47 g/mL vs. ~1.40 g/mL for Pichia and SEC mutant). These data again imply that the glycosylation pattern of Pichia-and Saccharomyces-produced invertase differ.

d. N-Terminal Analysis

The amino terminal sequence of purified recombinant invertase was determined using automated Edman degradation. Approximately 100 picomoles of protein was sequenced on a gas-phase sequencer (Applied Biosystems).

The sequence of GS115/pGS102-produced invertase yielded the expected N-terminal amino acid sequence, thus demonstrating the synthesis of authentic, correctly processed invertase.

EXAMPLE V

Growth of Sucrose Competent Pichia in Chemostat

Chemostat runs were performed using a Phillips designed two liter continuous fermentor equipped with monitors and controls for pH, dissolved oxygen (DO), agitator speed, temperature and airflow. Feed addition was achieved with a Milton-Roy Minipump. Feed was sterilized by autoclaving and maintained by filtration (Pall Ultipor Disposable Filter Assembly DFA 4001AR, 0.2 micron absolute). Each run was maintained at pH 4.0 by addition of $NH_3$ gas into the air stream. Temperature was held at 30°C. The dissolved oxygen concentration ranged from 45% to 80% air saturation. Chemostat working volume ranged from 1.3 to 2.1 liters. Cell density, productivity, and yields were determined from washed cell dry weights.

Each chemostat run was made under steady-state conditions where the dilution rate (d) was equal to the growth rate of the culture. Thus, the growth rate was controlled by adjusting the flow of fresh medium into the fermentor. Typical values for D ranged from 0.143 $hr^{-1}$ to 0.067 $hr^{-1}$.

Chemostat cultures were started with 5% inocula freshly grown in 100 mL of MM medium and sparged with 600 mL/min of filtered air. After depletion of the initial carbon source in the start-up medium, 1X FM-21 supplemented with 10% of the appropriate carbon source was pumped through the chemostat with a D value of 0.1 $hr^{-1}$. A 70% dissolved oxygen concentration was maintained by increasing airflow and supplementing with pure $O_2$ as required. A constant pH of 4.0 was maintained with periodic additions of $NH_3$. When the culture reached steady state and a cell density of about 50 g/L, the feed was switched to 2X FM-21 supplemented with 20% carbon source and allowed to reach steady state and a cell density of about 100 g/L. Finally, the feed was switched to 3X FM-21 supplemented with 30% carbon source and allowed to reach steady state. Then the feed pump rate and, therefore, the culture growth rate were increased to a D value of approximately 0.14 $hr^{-1}$ (corresponding to a generation time of about seven hours) and measurements of cell density, production rate and yield were made. Sucrose and glucose concentrations in the chemostate output were measured using Diastix glucose oxidase reagent strips (Miles Laboratories, Inc.).

## Analyses of Suc[+] *Pichia* Samples from High Cell Density Sucrose-chemostat

Cells obtained after growth of Suc[+]-*Pichia* strains in a sucrose chemostat to high cell density were analyzed to determine, (i) what fraction of cells retained the Suc[+] phenotype and, (ii) the status of *SUC2* gene (i.e., whether autonomous or chromosomally integrated). In the case of the chemostat samples of the NRRL Y-11430/Suc[+] 3-14 construct, it was found that the frequency of Suc[+] cells increased from an initial value of 1 per 30,000 to a value of 1 per 1,500 at the end of two cycles of growth in the chemostat. See Table II below.

### TABLE II
### Stability of NRRL Y-11430/Suc[+] Phenotype*

| Sample Source | Suc[+]/ Total Cells | % Suc[+] | Status of pTSU1 |
|---|---|---|---|
| First Sucrose Chemostat: | | | |
| 24h | 1/25,000 | 0.004 | ND** |
| 48h | 1/11,000 | 0.009 | Integrated |
| 120h (final sample) | 1/6000 | 0.017 | Integrated |
| Second Sucrose Chemostat: | | | |
| Final Sample | 1/1500 | 0.07 | Integrated |
| 72 h in Shake Flask on: | | | |
| MSu (1% Sucrose) | 1/1000 | 0.1 | ND |
| MD (1% Dextrose) | 1/100,000 | 0.001 | ND |
| Low MSu | | | Integrated |
| (0.05% Sucrose) | 1/50 | 2 | +Autonomous |

\* 11430/Suc[+] 3-14 transformants grown under different conditions, were diluted, and aliquots plated on MSu to determine the percent of cells retaining the Suc[+] phenotype. The total number of cells plated was determined by plating dilutions on MD plates. An aliquot of the final sample from the first chemostat cycle served as the inoculum for the second chemostat cycle. In shake flask experiments, similar results were obtained with all the three other Suc[+] 11430-SC5 transformants (data not shown). Only one representative transformant (Suc[+] 3-14) was tested in the chemostat.

\*\* ND: Not determined.

Southern hybridization analysis was also carried out on these samples, which analysis indicated that the *SUC2* gene was also present in association with chromosomal DNA. Because this construct performed well in the chemostat, it is presently believed that a small percent (~0.07%) of Suc[+] cells in the population will cause sufficient extracellular breakdown of sucrose to permit other cells to grow optimally. Under these conditions of growth, it may not be possible to enrich for Suc[+] cells any further.

The examples have been provided merely to illustrate the practice of the invention and should not be read so as to limit the scope of the invention or the appended claims in any way. Reasonable variations and modifications, not departing from the essence and spirit of the invention, are contemplated to be within the scope of patent protection desired and sought.

The following pages 29 to 36 of the description contain preferred embodiments 1 to 30.

1. Method for producing a desired protein in secreted form in yeast, wherein said desired protein has a pattern of glycosylation substantially similar to the high mannose-type mammalian protein pattern of glycosylation, which method comprises expressing a coding sequence coding for the desired protein in a host cell of the genus *Pichia* under the control of a promoter and a secretion signal.

2. Method in accordance with embodiment 1 wherein the pattern of glycosylation imparted to said desired protein comprises an average of 8-14 mannose units per glycosylation site.

3. Method in accordance with embodiment 2 wherein at least 80%, but not greater than 88% of the sugar units employed for glycosylation at each glycosylation site are mannose units.

4. Method in accordance with embodiment 1 wherein said protein having a high mannose-type mammalian pattern of glycosylation is selected from the group consisting of:

HTLV-III env protein,

chicken ovalbumin,

Sindbis virus proteins,

bovine rhodopsin,

human immunoglobulins, and

viral antigens.

5. Method in accordance with embodiment 1 wherein said secretion signal is selected from the group consisting of:

the *S. cerevisiae* invertase secretion signal,

the *S. cerevisiae* acid phosphatase secretion signal,

the *S. cerevisiae* alpha-mating factor secretion signal, and

the *Pichia pastoris* acid phosphatase secretion signal.

6. Method in accordance with embodiment 5 wherein said invertase secretion signal is 57 base pairs in length and has the following nucleotide sequence:

ATG-CTT-TTG-CAA-GCT-TTC-CTT-TTC-CTT-TTG-

GCT-GGT-TTT-GCA-GCC-AAA-ATA-TCT-GCA-.

7. Method in accordance with embodiment 5 wherein said invertase secretion signal comprises the following amino acid sequence:

Met-Leu-Leu-Gln-Ala-Phe-Leu-Phe-Leu-Leu-

Ala-Gly-Phe-Ala-Ala-Lys-Ile-Ser-Ala-.

8. Method in accordance with embodiment 1 wherein said promoter is capable of controlling the transcription of messenger RNA when positioned at the 5′ end of the secretion signal; wherein said secretion signal is positioned at the 5′ end of the polypeptide encoding region; wherein said promoter is responsive to at least one of the conditions selected from the group consisting of:

(i) the presence of methanol in the culture medium with which a host organism containing said DNA fragment is in contact,

(ii) the presence of a non-catabolite repressing carbon source other than methanol in the culture medium with which a host organism containing said DNA fragment is in contact, and

(iii) carbon source starvation in the culture medium with which a host organism containing said DNA fragment is in contact after growth of the host organism on a catabolite repressing carbon and energy source.

9. Method in accordance with embodiment 1 wherein said promoter is selected from the group consisting of:

the *Pichia* AOX1 regulatory region,

the *Pichia* DAS1 regulatory region,

the *Pichia* glyceraldehyde-3-phosphate dehydrogenase regulatory region,

the *Pichia* acid phosphatase regulatory region,

the *Saccharamyces* acid phosphatase regulatory region,

the *Saccharomyces* alpha-mating factor regulatory region,

the *Saccharomyces* glyceraldehyde-3-phosphate dehydrogenase regulatory region, and

the *Pichia* p40 regulatory region.

10. Method in accordance with embodiment 1 wherein said expressing of said coding sequence is accomplished by cultivating said host yeast cell of the genus *Pichia* under conditions under which the promoter sequence is activated for expression of the polypeptide coding sequence.

11. Method in accordance with embodiment 1 wherein said host cell is selected from the group consisting of:

*Pichia* NRRL Y-15851 (GTS115),

*Pichia* NRRL Y-18014 (GS190),

*Pichia* NRRL Y-18017 (PPF1),

*Pichia* NRRL Y-11430, and

*Pichia* NRRL Y-11431.

12. Process for the growth of yeast strains on substrates which said yeast strains are normally incapable of utilizing as a carbon and energy source, which process comprises:

(a) transforming said yeast strains with a DNA fragment encoding the gene function which the wild-type yeast strain lacks,

(b) regenerating the transformants obtained as a result of step (a) on non-selective regeneration medium,

(c) subjecting the regenerated transformants produced in accordance with step (b) to selective growth conditions whereby only those colonies which have incorporated the gene function encoded by said DNA fragment are capable of growth,

(d) selecting those regenerated transformants which grow under the conditions set forth in step (c), and

(e) growing the regenerated transformants selected in accordance with step (d) on said substrate which *Pichia* is incapable of utilizing as a carbon and energy source.

13. A process in accordance with embodiment 12 wherein said yeast strain in selected from the genera consisting of:

*Pichia, Saccharomyces, Kluyveromyces, Candida, Torulopsis, Hansenula, Schizosaccharomyces,*

*Citeromyces, Pachysolen Debaromyces, Metschunikowia, Rhodosporidium Leucosporidium,*

*Botryoascus, Sporidiobolus, Endomycopsis*, and the like.

14. A process in accordance with embodiment 12 wherein said yeast strain is selected from the genus *Pichia*.

15. A process in accordance with embodiment 12 wherein said DNA fragment is selected from the group consisting of:

the invertase gene,

the α-galactosidase gene,

the β-galactosidase and lactose permease genes, and

the β-glucosidase gene.

16. A process in accordance with embodiment 14 wherein said DNA fragment encodes a protein with the ability to promote the hydrolysis of sucrose to fructose and glucose.

17. A process in accordance with embodiment 16 wherein said DNA fragment is at least a portion of the invertase gene isolated from a strain of *Saccharomyces cerevisiae*.

18. A process in accordance with embodiment 14 wherein said yeast strains of the genus *Pichia* are prototrophic strains.

19. A process in accordance with embodiment 18 wherein said prototrophic strains of *Pichia* are members of the genus *Pichia pastoris*.

20. A process in accordance with embodiment 19 wherein said prototrophic strain is *Pichia pastoris* NRRL Y-11430.

21. A process in accordance with embodiment 14 wherein said yeast strain of the genus *Pichia* is selected from the group consisting of:

*Pichia pastoris* NRRL Y-11430,

*Pichia pastoris* NRRL Y-11431,

*Pichia pastoris* NRRL Y-15851,

*Pichia pastoris* NRRL Y-18014, and

*Pichia pastoris* NRRL Y-18017.

22. A process in accordance with embodiment 16 wherein said carbon and energy source is sucrose.

23. A serially arranged DNA fragment comprising:

(a) a regulatory region which is capable of controlling the transcription of messenger RNA when positioned at the 5′ end of a secretion signal and polypeptide encoding region; wherein said regulatory region is responsive to at least one of the conditions selected from the group consisting of:

(i) the presence of methanol in the culture medium with which a host organism containing said DNA fragment is in contact,

(ii) the presence of a non-catabolite repressing carbon source other than methanol in the culture medium with which a host organism containing said DNA fragment is in contact, and

(iii) carbon source starvation in the culture medium with which a host organism containing said DNA fragment is in contact after growth of the host organism on a catabolite repressing carbon and energy source,

(b) a yeast secretion signal, and

(c) a coding sequence which codes for a protein which is biologically active when said protein has a high mannose-type mammalian protein glycosylation pattern.

24. A DNA fragment in accordance with embodiment 23 whrein said secretion signal is selected from the group consisting of:

the *S. cerevisiae* invertase secretion signal,

the *S. cerevisiae* acid phosphatase secretion signal,

the *S. cerevisiae* alpha-mating factor secretion signal, and

the *Pichia pastoris* acid phosphatase secretion signal.

25. A DNA fragment in accordance with embodiment 24 wherein said invertase secretion signal is 57 base pairs in length and has the following nucleotide sequence:

ATG-CTT-TTG-CAA-GCT-TTC-CTT-TTC-CTT-TTG-GCT-GGT-TTT-GCA-GCC-AAA-ATA-TCT-GCA-.

26. A DNA fragment in accordance with embodiment 24 wherein said invertase secretion signal comprises the following amino acid sequence:

Met-Leu-Leu-Gln-Ala-Phe-Leu-Phe-Leu-Leu-Ala-Gly-Phe-Ala-Ala-Lys-Ile-Ser-Ala-.

27. A serially arranged DNA fragment comprising:

(a) a promoter selected from the group consisting of:

the *Pichia* AOX1 regulatory region,

the *Pichia* DAS1 regulatory region,

the *Pichia* glyceraldehyde-3-phosphate dehydrogenase regulatory region,

the *Pichia* acid phosphatase regulatory region,

the *Saccharomyces* acid phosphatase regulatory region,

the *Saccharomyces* alpha-mating factor regulatory region,

the *Saccharomyces* glyceraldehyde-3-phosphate dehydrogenase regulatory region, and

the *Pichia* p40 regulatory region,

(b) a yeast secretion signal, and

(c) a coding sequence which codes for a protein which is biologically active when said protein has a pattern of glycosylation substantially similar to the high mannose-type mammalian protein glycosylation pattern.

28. A DNA fragment in accordance with embodiment 27 wherein said secretion signal is selected from the group consisting of:

the *S. cerevisiae* invertase secretion signal,

the *S. cerevisiae* acid phosphatase secretion signal,

the *S. cerevisiae* alpha-mating factor secretion signal, and

the *Pichia pastoris* acid phosphatase secretion signal.

29. A DNA fragment in accordance with embodiment 28 wherein said invertase secretion signal is 57 base pairs in length and has the following nucleotide sequence:

ATG-CTT-TTG-CAA-GCT-TTC-CTT-TTC-CTT-TTG-GCT-GGT-TTT-GCA-GCC-AAA-ATA-TCT-GCA-.

30. A DNA fragment in accordance with embodiment 28 wherein said invertase secretion signal comprises the following amino acid sequence:

Met-Leu-Leu-Gln-Ala-Phe-Leu-Phe-Leu-Leu-Ala-Gly-Phe-Ala-Ala-Lys-Ile-Ser-Ala-.

## Claims

1. A method for producing a desired protein in secreted form in yeast, wherein said desired protein has a pattern of glycosylation substantially similar to the high mannose-type mammalian protein pattern of glycosylation, **characterized by** expressing a coding sequence coding for the desired protein in a host cell of the genus Pichia under the control of a promoter and a secretion signal.

2. The method of claim 1 characterized in that the pattern of glycosylation imparted to said desired protein comprises an average of 8-14 mannose units per glycosylation site; in particular wherein at least 80%, but not more than 88% of the sugar units employed for glycosylation at each glycosylation site are mannose units.

3. The method of claim 1 or 2 characterized in that said protein having a high mannose-type mammalian pattern of glycosylation is selected from HTLV-III env protein, chicken ovalbumin, Sindbis virus proteins, bovine rhodopsin, human immunoglobulins, and viral antigens; in particular wherein said secretion signal is selected from the S. cerevisiae invertase secretion signal, the S. cerevisiae acid phosphatase secretion signal, the S. cerevisiae alpha-mating factor secretion signal, and the Pichia pastoris acid phosphatase secretion signal; in particular wherein said invertase secretion signal is 57 base pairs in length and has the following nucleotide sequence:

ATG-CTT-TTG-CAA-GCT-TTC-CTT-TTC-CTT-TTG-GCT-GGT-TTT-GCA-GCC-AAA-ATA-TCT-GCA-;

in particular wherein said invertase secretion signal comprises the following amino acid sequence:

Met-Leu-Leu-Gln-Ala-Phe-Leu-Phe-Leu-Leu-Ala-Gly-Phe-Ala-Ala-Lys-Ile-Ser-Ala-.

4. The method of any of the preceding claims characterized in that said promoter is capable of controlling the transcription of messenger RNA when positioned at the 5' end of the secretion signal; wherein said secretion signal is positioned at the 5' end of the polypeptide encoding region; wherein said promoter is responsive to at least one of the following conditions:

(i) the presence of methanol in the culture medium with which a host organism containing said DNA fragment is in contact,

(ii) the presence of a non-catabolite repressing carbon source other than methanol in the culture medium with which a host organism containing said DNA fragment is in contact, and

(iii) carbon source starvation in the culture medium with which a host organism containing said DNA fragment is in contact after growth of the host organism on a catabolite repressing carbon and energy source;

in particular wherein said promoter is selected from

the Pichia AOX1 regulatory region,

the Pichia DAS1 regulatory region,

the Pichia glyceraldehyde-3-phosphate dehydrogenase regulatory region,

the Pichia acid phosphatase regulatory region,

the Saccharomyces acid phosphatase regulatory region,

the Saccharomyces alpha-mating factor regulatory region,

the Saccharomyces glyceraldehyde-3-phosphate dehydrogenase regulatory region, and

the Pichia p40 regulatory region;

in particular wherein said expressing of said coding sequence is accomplished by cultivating said host yeast cell of the genus Pichia under conditions under which the promoter sequence is activated for expression of the polypeptide coding sequence;

in particular wherein said host cell is selected from Pichia NRRL Y-15851 (GTS115), Pichia NRRL Y-18014 (GS190), Pichia NRRL Y-18017 (PPF1), Pichia NRRL Y-11430, and Pichia NRRL Y-11431.

5. Process for the growth of yeast strains on substrates which said yeast strains are normally incapable of utilizing as a carbon and energy source, characterized by

(a) transforming said yeast strains with a DNA fragment endcoing the gene function which the wild-type yeast strain lacks,

(b) regenerating the transformants obtained as a result of step (a) on non-selective regeneration medium,

(c) subjecting the regenerated transformants produced in accordance with step (b) to selective growth conditions whereby only those colonies which have incorporated the gene function encoded by said DNA fragment are capable of growth,

(d) selecting those regenerated transformants which grow under the conditions set forth in step (c), and

(e) growing the regenerated transformants selected in accordance with step (d) on said substrate which Pichia is incapable of utilizing as a carbon and energy source.

6. The process of claim 5 characterized in that said yeast strain is selected from the genera Pichia, Saccharomyces, Kluyveromyces, Candida, Torulopsis, Hansenula, Schizo saccharomyces, Citeromyces, Pachysolen, Debaromyces, Metschunikowia, Rhodosporidium, Leucosporidium, Botryoascus, Sporidiobolus, and Endomycopsis; in particular wherein said yeast strain is selected from the genus Pichia; in particular wherein said yeast strain of the genus Pichia is selected from Pichia pastoris NRRL Y-11430, Pichia pastoris NRRL Y-11431, Pichia pastoris NRRL Y-15851, Pichia pastoris NRRL Y-18014, and Pichia pastoris NY-18017, in particular wherein said yeast strains of the genus Pichia are prototrophic strains; in particular wherein said prototrophic strains of Pichia are members of the genus Pichia pastoris; is particular wherein said prototrophic strain is Pichia pastoris NRRL Y-11430.

7. The process of claim 5 or 6 characterized in that said DNA fragment is selected from the invertase gene, the α-galactosidase gene, the β-galactosidase and lactose permease genes, and the β-glucosidase gene; in particular wherein said DNA fragment encodes a protein with the ability to promote the hydrolysis of sucrose to fructose and glucose; in particular wherein said carbon and energy source is sucrose; in particular wherein said DNA fragment is at least a portion of the invertase gene isolated from a strain of Saccharomyces cerevisiae.

8. A serially arranged DNA frament comprising:

(a) a regulatory region which is capable of controlling the transcription of messenger RNA when positioned at the 5′ end of a secretion signal and polypeptide encoding region; wherein said regulatory region is responsive to at least one of the following conditions:

(i) the presence of methanol in the culture medium with which a host organism containing said DNA fragment is in contact,

(ii) the presence of a non-catabolite repressing carbon source other than methanol in the culture medium with which a host organism containing said DNA frag ment is in contact, and

(iii) carbon source starvation in the culture medium with which a host organism containing said DNA fragment is in contact after growth of the host organism on a catabolite repressing carbon and energy source,

(b) a yeast secretion signal, and

(c) a coding sequence which codes for a protein which is biologically active when said protein has a high mannose-type mammalian protein glycosylation pattern.

9. The DNA fragment of claim 8 characterized in that said secretion signal is selected from
the S. cerevisiae invertase secretion signal,
the S. cerevisiae acid phosphatase secretion signal,
the S. cerevisiae alpha-mating factor secretion signal, and
the Pichia pastoris acid phosphatase secretion signal;
in particular wherein said invertase secretion signal is 57 base pairs in length and has the following nucleotide sequence:
    ATG-CTT-TTG-CAA-GCT-TTC-CTT-TTC-CTT-TTG-GCT-GGT-TTT-GCA-GCC-AAA-ATA-TCT-GCA-;
in particular wherein said invertase secretion signal comprises the following amino acid sequence:
    Met-Leu-Leu-Gln-Ala-Phe-Leu-Phe-Leu-Leu-Ala-Gly-Phe-Ala-Ala-Lys-Ile-Ser-Ala-.

10. A serially arranged DNA frament comprising:

(a) a promoter selected from
the Pichia AOX1 regulatory region,
the Pichia DAS1 regulatory region,
the Pichia glyceraldehyde-3-phosphate dehydrogenase regulatory region,
the Pichia acid phosphatase regulatory region,
the Saccharomyces acid phosphatase regulatory region,
the Saccharomyces alpha-mating factor regulatory region,
the Saccharomyces glyceraldehyde-3-phosphate dehydrogenase regulatory region, and
the Pichia p40 regulatory region,

(b) a yeast secretion signal, and

(c) a coding sequence which codes for a protein which is biologically active when said protein has a pattern of glycosylation substantially similar to the high mannose-type mammalian protein glycosylation pattern.

11. The DNA fragment of claim 10 characterized in that said secretion signal is selected from
the S. cerevisiae invertase secretion signal,
the S. cerevisiae acid phosphatase secretion signal,
the S. cerevisiae alpha-mating factor secretion signal, and
the Pichia pastoris acid phosphatase secretion signal;
in particular wherein said invertase secretion signal is 57 base pairs in length and has the following nucleotide sequence:
    ATG-CTT-TTG-CAA-GCT-TTC-CTT-TTC-CTT-TTG-GCT-GGT-TTT-GCA-GCC-AAA-ATA-TCT-GCA-;
in particular wherein said invertase secretion signal comprises the following amino acid sequence:
    Met-Leu-Leu-Gln-Ala-Phe-Leu-Phe-Leu-Leu-Ala-Gly-Phe-Ala-Ala-Lys-Ile-Ser-Ala.

FIG. 1

FIG. 2

FIG. 3

# FIG. 4

# FIG. 5

pSEY303          LINKER

pGS100          pBSAGl5l   (NRRL B-18021)

pGS101          pYJ30 (NRRL B-15890)

pGSl02

FIG. 6

FIG. 7